# EUROPEAN PATENT APPLICATION

(11) **EP 2 668 941 A1**
(43) Date of publication of application: **04.12.2013**
(21) Application number: 12382221.5
(22) Date of filing: 31.05.2012
(51) Int. Cl.: A61K 9/14, A61K 31/4706, A61K 9/00, A61P 11/08, A61P 11/06

(54) **Novel dosage form and formulation of abediterol**

(71) Applicant: Almirall, S.A., 08022 Barcelona (ES)
(72) Inventor: Allain Ruiz, Sandrine, 08980 Sant Feliu de Llobregat (ES); Seoane Nuñez, Beatriz, 08980 Sant Feliu de Llobregat (ES); De Miquel Serra, Gonzalo, 08980 Sant Feliu de Llobregat (ES)
(74) Representative: Elzaburu Marquez, Alberto

(57) **Abstract**

The present invention provides a pharmaceutical composition in the form of a dry powder for inhalation comprising abediterol or a pharmaceutically acceptable salt in admixture with a pharmaceutically acceptable carrier, providing upon inhalation a dose equivalent to a metered nominal dose of about 1.25 or about 2.5 micrograms of free base abediterol administered with the Genuair® inhaler. The present invention also provides said pharmaceutical composition for use in the treatment of respiratory disease such as asthma and chronic obstructive pulmonary disease COPD.

## Description

### Field of the Invention

The present invention relates to a novel dosage for abediterol and to novel methods and formulations for the treatment of respiratory disease in a mammal, especially asthma and chronic obstructive pulmonary disease (COPD), using abediterol.

### Background of the Invention

Abediterol [5-(2-{[6-(2,2-difluoro-2-phenylethoxy)hexyl]amino}-1(R)-hydroxyethyl)-8-hydroxyquinolin-2(1H)-one] is described in WO 2006/122788, WO 2008 /095720 and WO 2010/072354. It has the structure shown below.

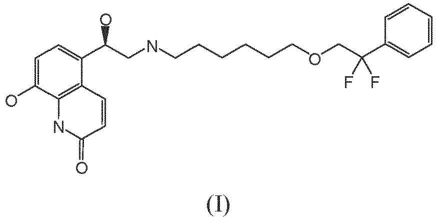

Abediterol is a potent, long acting and selective β2 adrenergic receptor agonist intended for administration by inhalation for treatment of respiratory diseases, especially asthma and COPD, currently in clinical trials.

Dry powder inhalers are well known devices for administering pharmaceutically active agents to the respiratory tract. They are particularly suitable for the administration of active agents in the treatment of respiratory diseases such as asthma, COPD, emphysema or the like.

The Genuair® inhaler is a novel, breath-actuated multidose, dry powder inhaler designed for the effective delivery of various types of inhaled drugs (Greguletz et al., Am. J. Respir. Crit. Care Med., 2009, 179,:A4578). This inhaler, which has been developed from the Novolizer® inhaler (WO 97/000703) incorporates a number of technological advances including improved fluid and particle dynamics to ensure powder entrainment during inhalation and affective deagglomeration of the inhalation powder into a suitable aerosol even at low inhalation flow rate and volume (WO 03/000325 and W02006/008027). In addition the Genuair® inhaler has been used in clinical trials to reliably deliver various types of drugs as both monotherapy and combination therapy (H. Chrystyn et al., Int. J. Clinical Practice, 66, 3, 309-317, 2012).

### Summary of the Invention

It has now been discovered that for the treatment of respiratory disorders in humans, particularly asthma and COPD, abediterol is most effective upon inhalation of a dosage equivalent to about 1.25 or to about 2.5 micrograms metered nominal dose of the free base administered with the Genuair® inhaler.

When administered with the Genuair® inhaler a metered nominal dose of about 1.25 micrograms of free base abediterol is equivalent to about 1.1 micrograms delivered dose and to about 0.45 micrograms fine particle dose.

In a similar way a metered nominal dose of about 2.5 micrograms of free base abediterol administered with the Genuair® inhaler is equivalent to about 2.3 micrograms delivered dose and to about 1.0 micrograms fine particle dose.

The invention thus provides in a first embodiment a pharmaceutical composition in the form of a dry powder for inhalation comprising abediterol or a pharmaceutically acceptable salt thereof in admixture with a pharmaceutically acceptable carrier, providing upon inhalation a dose equivalent to a metered nominal dose of about 1.25 or about 2.5 micrograms of free base abediterol administered with the Genuair® inhaler.

This pharmaceutical composition can be provided as a single dose formulation or as a multidose formulation. In both cases the inhaler devices are calibrated to provide a dose equivalent to a metered nominal dose of about 1.25 or about 2.5 micrograms of free base abediterol administered with the Genuair® inhaler. This composition can be administered one or more times per day, preferably once or twice a day, most preferably once a day.

The invention also provides a method of treating a respiratory condition, e.g., selected from asthma and chronic obstructive pulmonary disease, in a patient in need of such treatment, comprising administering a pharmaceutical composition, according to the previous paragraph and providing a dose of abediterol equivalent to a metered nominal dose of about 1.25 or about 2.5 micrograms of free base abediterol administered with the Genuair® inhaler. The invention further provides the use of abediterol in the manufacture of a pharmaceutical composition as described in the preceding paragraph, for use in such a method.

Abediterol may be administered as monotherapy, or in combination with one or more additional anti-inflammatory and/or bronchodilating agents, e.g., corticosteroids, such as budesonide, mometasone or fluticasone, PDE IV inhibitors, such as roflumilast or cilomilast and M3 antimuscarinic agents, such as, glycopyrronium, tiotropium or aclidinium. The invention thus also provides pharmaceutical compositions as described above further comprising such an additional agent, as well as methods of treating respiratory disorders comprising administering these compositions.

### Detailed description of the invention

Typically, the present invention provides a pharmaceutical composition in the form of a dry powder for inhalation comprising abediterol or a pharmaceutically acceptable salt thereof in admixture with a pharmaceutically acceptable carrier, providing upon inhalation a dose equivalent to a metered nominal dose of about 1.25 micrograms of free base abediterol administered with the Genuair® inhaler.

Typically, the present invention also provides a pharmaceutical composition in the form of a dry powder for inhalation comprising abediterol or a pharmaceutically acceptable salt thereof in admixture with a pharmaceutically acceptable carrier, providing upon inhalation a dose equivalent to a metered nominal dose of about 2.5 micrograms of free base abediterol administered with the Genuair® inhaler.

The term "therapeutically effective amount" refers to an amount sufficient to effect treatment when administered to a patient in need of treatment.

The term "metered nominal dose" refers to the quantity of drug substance contained in the metering chamber of the delivery device and is normally expressed as quantity per inhalation.

Upon actuation, the drug substance leaves the device and becomes available to the patient as a so-called "delivered dose" (also known as "emitted dose"), which is normally smaller than the metered nominal dose, due to the mechanics of the device. Thus, the delivered dose is the amount of the drug which is available at the mouth for inhalation. The delivered dose can be measured using standard techniques known to those skilled in the art.

The term "fine particle dose" refers to the quantity of drug substance in the delivered dose below a cut off aerodynamic threshold of 5 micrometer, i.e. the delivered dose with an aerodynamic particle diameter less than 5 micrograms that is available for deposition in the lungs. The fine particle dose can be measured using standard techniques known to those skilled in the art.

In the context of dosage of an active agent, "about" as used herein means within the normal limits of acceptable variations as defined by the European and US Pharmacopeia of plus/minus 35% or preferably acceptable variations as defined by the current most stringent requirement, the US FDA draft guidance for inhaler of plus/minus 25% or the acceptable variations defined by the CHMP Guideline on the Pharmaceutical Quality of Inhalation and Nasal Products of plus/minus 15%.

Thus the delivered dose of "about 1.1 micrograms" is meant a target delivered dose ranging from 0.71 to 1.49 micrograms, preferably from 0.82 to 1.38 micrograms, more preferably from 0.93 to 1.27 micrograms. Similarly the delivered dose of "about 2.3 micrograms" is meant a target delivered dose ranging from 1.49 to 3.11 micrograms, preferably from 1.72 to 2.88, more preferably from 1.95 to 2.65.

Similarly, the fine particle dose is also subjected to the same variation and proportional to the delivered dose. Thus a delivered dose of "about 1.1 micrograms" corresponds to a fine particle dose of "about 0.45 micrograms" i.e. ranging from 0.29 to 0.61 micrograms, preferably from 0.33 to 0.57 micrograms, more preferably from 0.38 to 0.52 micrograms. The delivered dose of "about 2.3 micrograms" corresponds to a fine particle dose of about 1.0 micrograms, i.e. ranging from 0.65 to 1.35 micrograms, preferably from 0.75 to 1.25 micrograms, more preferably from 0.85 to 1.15 micrograms.

In the same way, a metered nominal dose of "about 2.5 micrograms" is meant a target nominal dose ranging from 1.62 to 3.38 micrograms, preferably from 1.87 to 3.13 micrograms, more preferably from 2.12 to 2.88 micrograms. Similarly the metered nominal dose of "about 1.25 micrograms" meant a target nominal dose ranging from 0.81 to 1.69 micrograms, preferably from 0.93 to 1.57 micrograms, more preferably from 1.06-1.44 micrograms.

The invention thus provides in a further embodiment a pharmaceutical composition in the form of a dry powder for inhalation comprising abediterol or a pharmaceutically acceptable salt thereof in admixture with a pharmaceutically acceptable carrier, providing upon inhalation a delivered dose ranging from 0.71 to 1.49 micrograms, preferably from 0.82 to 1.38 micrograms, more preferably from 0.93 to 1.27 micrograms of free base abediterol.

In another embodiment, the invention provides a pharmaceutical composition in the form of a dry powder for inhalation comprising abediterol or a pharmaceutically acceptable salt thereof in admixture with a pharmaceutically acceptable carrier, providing upon inhalation a delivered dose ranging from 1.49 to 3.11 micrograms, preferably from 1.72 to 2.88, more preferably from 1.95 to 2.65 of free base abediterol.

In a still another embodiment, the invention provides a pharmaceutical composition in the form of a dry powder for inhalation comprising abediterol or a pharmaceutically acceptable salt thereof in admixture with a pharmaceutically acceptable carrier, providing upon inhalation a fine particle dose ranging from 0.29 to 0.61 micrograms, preferably, from 0.33 to 0.57 micrograms being most preferably from 0.38 to 0.52 micrograms of free base abediterol.

The invention also provides in a further embodiment a pharmaceutical composition in the form of a dry powder for inhalation comprising abediterol or a pharmaceutically acceptable salt thereof in admixture with a pharmaceutically acceptable carrier, providing upon inhalation a fine particle dose ranging from 0.65 to 1.35 micrograms, preferably, from 0.75 to 1.25 micrograms being most preferably from 0.85 to 1.15 micrograms of free base abediterol.

In another embodiment, the invention provides a pharmaceutical composition in the form of a dry powder for inhalation comprising abediterol or a pharmaceutically acceptable salt thereof in admixture with a pharmaceutically acceptable carrier, providing upon inhalation a metered nominal dose ranging from 0.81 to 1.69 micrograms, preferably from 0.93 to 1.57 micrograms, more preferably from 1.06 to 1.44 micrograms of free base abediterol.

The invention also provides a pharmaceutical composition in the form of a dry powder for inhalation comprising abediterol or a pharmaceutically acceptable salt thereof in admixture with a pharmaceutically acceptable carrier, providing upon inhalation a metered nominal dose ranging from 1.62 to 3.38 micrograms, preferably from 1.87 to 3.13 micrograms, more preferably from 2.12 to 2.88 micrograms of free base abediterol. In a further embodiment, the present invention provides a pharmaceutical composition in the form of a dry powder for inhalation comprising abediterol or a pharmaceutically acceptable salt thereof in admixture with a pharmaceutically acceptable carrier, providing upon inhalation a dose equivalent to a metered nominal dose of about 0.625 micrograms of free base abediterol administered with the Genuair® inhaler. A dose of 0.625g abediterol has surprisingly been found to be the minimum dose having similar therapeutic effect to other β2 adrenergic receptor agonist compounds, such as salbutamol and indacaterol.

When administered with the Genuair® inhaler a metered nominal dose of about 0.625 micrograms of free base abediterol is equivalent to about 0.562 micrograms delivered dose and to about 0.22 micrograms fine particle dose.

As mentioned before, the delivered dose is subjected within the normal limits of acceptable variations as defined above. Thus the delivered dose of "about 0.562 micrograms" is meant a target delivered dose ranging from 0.365 to 0.759 micrograms, preferably from 0.421 to 0.703 micrograms, more preferably from 0.477 to 0.647 micrograms.

Similarly, the fine particle dose of "about 0.22 micrograms" is meant a target dose ranging from 0.14 to 0.30 micrograms, preferably from 0.16 to 0.28 micrograms, more preferably from 0.18 to 0.26 micrograms.

Similarly a metered nominal dose of "about 0.625 micrograms" is also subjected to the same variation limits as defined above and thus the metered nominal dose of "about 0.625 micrograms" is meant a target nominal dose ranging from 0.406 to 0.844 micrograms, preferably from 0.468 to 0.782 micrograms, more preferably from 0.531 to 0.719 micrograms.

The invention thus provides in a further embodiment a pharmaceutical composition in the form of a dry powder for inhalation comprising abediterol or a pharmaceutically acceptable salt thereof in admixture with a pharmaceutically acceptable carrier, providing upon inhalation a delivered dose ranging from 0.365 to 0.759 micrograms, preferably from 0.421 to 0.703 micrograms, more preferably from 0.477 to 0.647 micrograms of free base abediterol.

In another embodiment, the invention provides a pharmaceutical composition in the form of a dry powder for inhalation comprising abediterol or a pharmaceutically acceptable salt thereof in admixture with a pharmaceutically acceptable carrier, providing upon inhalation a fine particle dose ranging from 0.14 to 0.30 micrograms, preferably from 0.16 to 0.28 micrograms, more preferably from 0.18 to 0.26 micrograms of free base abediterol.

In a still another embodiment, the invention provides a pharmaceutical composition in the form of a dry powder for inhalation comprising abediterol or a pharmaceutically acceptable salt thereof in admixture with a pharmaceutically acceptable carrier, providing upon inhalation a nominal dose ranging from 0.406 to 0.844 micrograms, preferably from 0.468 to 0.782 micrograms, more preferably from 0.531 to 0.719 micrograms of free base abediterol.

These pharmaceutical compositions can be provided as a single dose formulation or as a multidose formulation. In both cases the inhaler devices are calibrated to provide a dose of free base abediterol as defined above administered with the Genuair® inhaler.

The term "treatment" as used herein refers to the treatment of a disease or medical condition in a patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

Typically, abediterol is administered in the form a salt derived from pharmaceutically-acceptable acids include acetic, benzenesulphonic, benzoic, camphosulphonic, citric, ethanesulphonic, fumaric, gluconic, glutamic, hydrobromic, hydrochloric, lactic, maleic, malic, mandelic, methanesulphonic, mucic, naphthalene-1,5-disulphonic acid (napadisylate), nitric, pantothenic, phosphoric, succinic, sulphuric, tartaric, p-toluenesulphonic, xinafoic (1-hydroxy-2-naphthoic acid). Particularly preferred are salts derived from fumaric, hydrobromic, hydrochloric, acetic, sulphuric, methanesulphonic, naphthalene-1,5-disulphonic, xinafoic, and tartaric acids. Most preferred are salts derived from methanesulphonic and naphthalene-1,5-disulphonic acids.

Salts derived from naphthalene-1,5-disulphonic acid are typically mononapadisylate or heminapadisylate salts and pharmaceutically acceptable solvates thereof. Abediterol is preferably administered in the form of heminapadisylate salt having the hollowing chemical structure:

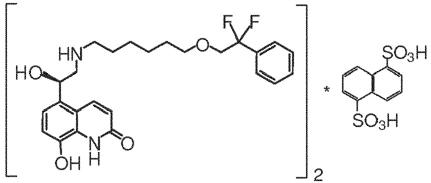

It is to be understood that all dose values of abediterol described in the present invention are related to the dose of abediterol as free base and thus the amount of the corresponding salt should be taken into account. Therefore, in the case that the salt used is the heminapadisylate, the metered nominal dose of 1.25 micrograms of abediterol free base as defined herein above will correspond to a metered nominal dose of about 1.64 micrograms of abediterol heminapadisylate. In the same way, the metered nominal dose of 2.5 micrograms as defined herein above will correspond to a metered nominal dose of about 3.28 micrograms of abediterol heminapadisylate and the metered nominal dose of 0.625 micrograms as defined herein above will correspond to a metered nominal dose of about 0.82 micrograms of abediterol heminapadisylate.

In case that the salt used is the mesylate, the metered nominal dose of 1.25 micrograms as defined herein above will correspond to a metered nominal dose of about 1.51 micrograms of abediterol mesylate. In the same way, the metered nominal dose of 2.5 micrograms as defined herein above will correspond to a metered nominal dose of about 3.02 micrograms of abediterol mesylate and the metered nominal dose of 0.625 micrograms as defined herein above will correspond to a metered nominal dose of about 0.755 micrograms of abediterol mesylate.

Abediterol is preferably administered in the form of a dry powder of a pharmaceutically acceptable salt, in admixture with a carrier, such as lactose powder, suitable for inhalation. For example, the abediterol is abediterol heminapadisylate in admixture with lactose powder.

The respiratory disease or condition to be treated with the formulations and methods of the present invention is typically asthma, acute or chronic bronchitis, emphysema, chronic obstructive pulmonary disease (COPD), bronchial hyperreactivity or rhinitis, in particular asthma or chronic obstructive pulmonary disease (COPD).

Typically, the pharmaceutical compositions of the present invention is suitable for administration by inhalation and may further comprise a therapeutically effective amount of one or more other therapeutic agents, as defined herein. However, any other form of topical, parenteral or oral application is possible. The application of inhaled dosage forms embodies the preferred application form, especially in the therapy of diseases or disorders of the lung.

The pharmaceutical composition of the present invention may be prepared by any of the methods well known in the art of pharmacy. All methods include the step of bringing the active ingredient(s) into association with the carrier. In general the pharmaceutical compositions are prepared by uniformly and intimately bringing into association the active ingredient(s) with liquid carriers or finely divided solid carriers or both and then, if necessary, shaping the product into the desired form.

The carrier for a pharmaceutical composition in the form of a dry powder is typically chosen from starch or a pharmaceutically acceptable sugar, such as lactose or glucose. Lactose is preferred.

Additional suitable carriers can be found in Remington: The Science and Practice of Pharmacy, 20th Edition, Lippincott Williams & Wilkins, Philadelphia, Pa., 2000.

The pharmaceutical compositions for inhalation are delivered with the help of inhalers, such as dry powder inhalers, aerosols or nebulisers. The inhaler is typically configured to deliver, upon actuation, a therapeutically effective amount of one or more other therapeutic agents, as defined herein.

Packaging of the compound of the invention in the inhaler may be suitable for unit dose or multi-dose delivery. In the case of multi-dose delivery, the compound of the invention can be pre-metered or metered in use. Dry powder inhalers are classified into three groups: (a) single dose, (b) multiple unit dose and (c) multi dose devices.

For inhalers of the first type (a), single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients. Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported.

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO 91/02558 and GB 2242134). They comprise the type of multiple unit dose inhalers (b) together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose devices (c) do not contain pre-measured quantities of the medicament containing powder. They consist of a relatively large container and a dose measuring principle that has to be operated by the patient. The container bears multiple doses that are isolated individually from the bulk of powder by volumetric displacement. Various dose measuring principles exist, including rotatable membranes (e.g. EP0069715) or disks (e.g. GB 2041763; EP 0424790; DE 4239402 and EP 0674533), rotatable cylinders (e.g. EP 0166294; GB 2165159 and WO 92/09322) and rotatable frustums (e.g. WO 92/00771), all having cavities which have to be filled with powder from the container. Other multi dose devices have measuring plungers with a local or circumferential recess to displace a certain volume of powder from the container to a delivery chamber or an air conduit (e.g. EP 0505321, WO 92/04068 and WO 92/04928), or measuring slides such as the Novolizer SD2FL (ex. Sofotec), also known as Genuair®, are described above.

Reproducible dose measuring is one of the major concerns for multi dose devices.

The powder formulation has to exhibit good and stable flow properties, because filling of the dose measuring cups or cavities is mostly under the influence of the force of gravity. For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeratian is obtained during discharge.

Consequently, separate disintegration means are necessary. However in practice, they are not always part of the inhaler design. Because of the high number of doses in multi-dose devices, powder adhesion onto the inner walls of the air conduits and the deagglomeration means must be minimized and/or regular cleaning of these parts must be possible, without affecting the residual doses in the device. Some multi dose inhalers have disposable drug containers that can be replaced after the prescribed number of doses has been taken (e.g. WO 97/000703). For such semi-permanent multi dose inhalers with disposable drug containers, the requirements to prevent drug accumulation are even stricter.

Apart from applications through dry powder inhalers the compositions of the invention can be administered in aerosols which operate via propellant gases or by means of so-called atomisers or nebulizers, via which solutions or suspensions of pharmacologically-active substances can be sprayed under high pressure so that a mist of inhalable particles results.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. To achieve these particle sizes the particles of the active ingredient as produced may be size reduced by conventional means, for example, by micronisation or supercritical fluid techniques. The desired fraction may be separated out by air classification or sieving. Preferably, the particles will be crystalline.

Achieving a high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient, for example a mono-, di- or polysaccharide or sugar alcohol, such as lactose, mannitol or glucose is generally employed. The particle size of the excipient will usually be much greater than the inhaled medicament within the present invention. When the excipient is lactose it will typically be present as lactose particles, preferably crystalline alpha lactose monohydrate, e.g., having an average particle size range of 20-1000 µm, preferably in the range of 90-150 µm. The average particle size can be measured using standard techniques known to those skilled in the art.

The median particle size approximately corresponds to the average and is the diameter where 50 mass-% of the particles have a larger equivalent diameter, and the other 50 mass-% have a smaller equivalent diameter. Hence the average particle size is generally referred to in the art as equivalent d50. The distribution of particle size around may affect flow properties, bulk density, etc. Hence to characterize a particle size diameter, other equivalent diameters can be used in addition to d50, such as d 10 and d90. d10 is the equivalent diameter where 10 mass-% of the particles have a smaller diameter (and hence the remaining 90% is coarser). d90 is the equivalent diameter where 90 mass-% of the particles have a smaller diameter. In one embodiment, the lactose particles for use in formulations of the invention have a d10 of 90 - 160 µm, a d50 of 170 ― 270 µm, and d90 of 290 ― 400 µm. The d10, d50 and d90 values can be measured using standard techniques known to those skilled in the art.

Suitable lactose materials for use in the present invention are commercially available, e.g., from DMV International (Respitose GR-001, Respitose SV-001, Respitose SV-003 or a mixture thereof), Meggle (Capsulac 60, Inhalac 70, Inhalac 120, Inhalac 230, Capsulac 60 INH, Sorbolac 400, or a mixture thereof), and Borculo Domo (Lactohale 100-200, Lactohale 200-300 and Lactohale 100-300, or a mixture thereof).

In another embodiment, the carrier used may be in the form of a mixture of different types of carrier having different particles sizes. For example, a mixture of a fine carrier and a coarse carrier may be present in the formulation, wherein the average particle size of the fine carrier is lower than the average particle size of the coarse carrier. Preferably the fine carrier may have an average particle size range of 1 - 50 µm, preferably 2 - 20 µm, more preferably, 5 ― 15 µm. The coarse carrier may have an average particle size range of 20 - 1000 µm, preferably 50-500 µm, more preferably 90-400 µm, being most preferably, 150-300 µm. The content of the fine carrier with respect to the coarse carrier may vary from 1% to 10%, preferably, from 3% to 6%, e.g., 5%, by weight of the total coarse carrier.

In one embodiment lactose particles for use in formulations of the invention is a mixture of a coarse lactose having a d10 of 90 - 160 µm, a d50 of 170 ― 270 µm, and d90 of 290 ― 400 µm and a fine lactose having a d10 of 2 - 4 µm, a d50 of 7 ― 10 µm, and d90 of 15 ― 24 µm.

The ratio by weight between the lactose particles and the abediterol, will depend on the inhaler device used, but is typically, e.g., 1000:1 to 40000:1, for example 2000:1 to 20000:1, e.g., 4000-10000:1.

In a preferred embodiment, the abediterol is administered in the form of a dry powder of a pharmaceutically acceptable salt in admixture with lactose, in a ratio by weight of the active ingredient to lactose of 1:20000 to 1:2000, suitable for administration via a dry powder inhaler, wherein the active ingredient particles have an average particle size of from 1.5 to 5 µm in diameter, e.g., less than 3 µm in diameter, and the lactose particles have a d10 of 90 - 160 µm, a d50 of 170 ― 270 µm, and d90 of 290 ― 400 µm. Said lactose particles are optionally mixed with a fine lactose having a particle size d10 of 2 - 4 µm, a d50 of 7 ― 10 µm, and d90 of 15 ― 24 µm.

Additional active agents such as M3 antagonists (anticholinergics), PDE IV inhibitors, corticosteroids, leukotriene D4 antagonists, inhibitors of egfr-kinase, p38 kinase inhibitors or NK1 receptor agonists may be utilized in the methods and formulations of the inventions. For example, the invention provides abediterol formulations as described herein further comprising an effective amount of such additional active agents, e.g. further comprising an effective amount of a M3 antagonist, a PDE IV inhibitor, or a corticosteroid. The invention also provides methods for treating respiratory conditions as herein before described, e.g., asthma or COPD, comprising administering an abediterol formulation as described herein and further comprising administering simultaneously effective amount of such additional active agents, e.g. further comprising an effective amount of a M3 antagonist, a PDE IV inhibitor, or a corticosteroid.

Examples of suitable M3 antagonists (anticholinergics) that can be combined with abediterol are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-y1]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable corticosteroids and glucocorticoids that can be combined with abediterol are prednisolone, methylprednisolone, dexamethasone, dexamethasone acetate, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone, mometasone furoate, rimexolone, prednisolone farnesylate, ciclesonide, butixocort propionate, RS-85095, CGP-13774, GW-250495, deltacortisone, NO-Prednisolone, NO-Budesonide, etiprednol dicloacetate, QAE-397, 7beta-OH-EPIA, RPR-106541, deprodone propionate, fluticasone, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, desisobutyrylciclesonide, hydrocortisone acetate, hydrocortisone sodium succinate, prednisolone sodium phosphate and hydrocortisone probutate, prednisolone sodium metasulfobenzoate and clobetasol propionate.

Examples of suitable PDE4 inhibitors that can be combined with abediterol are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, revamilast, ronomilast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, GSK-356278, TA-7906, CP-80633, RPL-554, NIK-616, GPD-1116, D4396, UK-500001, BLX-914, 2-carbomethoxy-4-cyano-4-(3-cyclopropylmethoxy-4-difluroromethoxyphenyl) cyclohexan1-one, cis [4-cyano-4-(3-cyclopropylmethoxy-4-difluoro methoxyphenyl) cyclohexan-1-ol, 5(S)-[3-(Cyclopentyloxy)-4-methoxyphenyl]-3(S)-(3-methylbenzyl) piperidin-2-one (IPL-455903), ONO-6126 (Eur Respir J 2003, 22(Suppl. 45): Abst 2557) and the compounds claimed in the PCT patent applications number WO 03/097613, WO 2004/058729, WO 2005/049581, WO 2005/123693, WO 2005/123692, and WO 2010/069504.

Particularly preferred additional therapeutic agents are selected from the group consisting oftiotropium, glycopyrronium, aclidinium, 1-(2-Phenylethyl)-3-(9H-xanthen-9-ylcarbonyloxy)-1-azoniabicyclo[2.2.2]octane, mometasone, fluticasone, budesonide, rolipram, roflumilast and cilomilast, more preferably, tiotropium, aclidinium, mometasone, fluticasone and roflumilast, in free or pharmaceutically acceptable salt form.

For example, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 1.25 or about 2.5 micrograms of free base abediterol together with a dose equivalent to a metered nominal dose of about 50-900 µg (e.g. 50, 70, 80, 85, 100, 110, 150, 170, 200, 220, 300, 330, 340, 350, 400, 440, 800, 880 or 900 µg) of mometasone in free or pharmaceutically acceptable salt form,administered with the Genuair® inhaler. Preferably the mometasone is mometasone furoate.

Thus in a preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 1.25 micrograms of free base abediterol together with a dose equivalent to a metered nominal dose of about 85 micrograms of mometasone furoate administered with the Genuair® inhaler.

The present invention also provides, in a preferred embodiment, a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 1.25 micrograms of free base abediterol together with a dose equivalent to a metered nominal dose of about 170 micrograms of mometasone furoate administered with the Genuair® inhaler.

In another preferred embodiment, the present invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 1.25 micrograms of free base abediterol together with a dose equivalent to a metered nominal dose of about 340 micrograms of mometasone furoate administered with the Genuair® inhaler.

Similarly, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 2.5 micrograms of free base abediterol together with a dose equivalent to a metered nominal dose of about 85 micrograms of mometasone furoate administered with the Genuair® inhaler.

The present invention also provides, in a preferred embodiment, a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 2.5 micrograms of free base abediterol together with a dose equivalent to a metered nominal dose of about 170 micrograms of mometasone furoate administered with the Genuair® inhaler.

In another preferred embodiment, the present invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 2.5 micrograms of free base abediterol together with a dose equivalent to a metered nominal dose of about 340 micrograms of mometasone furoate administered with the Genuair® inhaler.

When administered with the Genuair® inhaler a metered nominal dose of about 85 micrograms of mometasone furoate is equivalent to about 76 micrograms delivered dose and to about 30 micrograms fine particle dose. In a similar way a metered nominal dose of about 170 micrograms of mometasone furoate administered with the Genuair® inhaler is equivalent to about 150 micrograms delivered dose and to about 65 micrograms fine particle dose. Finally, a metered nominal dose of about 340 micrograms of mometasone furoate administered with the Genuair® inhaler is equivalent to about 300 micrograms delivered dose and to about 130 micrograms fine particle dose.

As mentioned before, the delivered dose of mometasone furoate is also subjected to the same acceptable variation limits as defined by the European and US pharmacopeia. Thus the delivered dose of "about 76 micrograms" is meant a target delivered dose ranging from 49.40 to 102.60 micrograms (plus/minus 35%), preferably from 57 to 95 micrograms (plus/minus 25%), more preferably from 64.6 to 87.4 micrograms (plus/minus 15%). In the same way, the delivered dose of "about 150 micrograms" is meant a target delivered dose ranging from 97.50 to 202.50 micrograms, preferably from 112.50 to 187.50 micrograms, more preferably from 127.50 to 172.50 micrograms. Similarly the delivered dose of "about 300 micrograms" is meant a target delivered dose ranging from 195 to 405 micrograms, preferably from 225 to 375 micrograms, more preferably from 255 to 345 micrograms.

As mentioned before the fine particle dose is the amount of drug substance in the delivered dose below a cut off aerodynamic threshold of 5 micrometer and thus is also subjected to the same acceptable variation limits and proportional to the delivered dose as defined by the European and US pharmacopeia.

Thus the fine particle dose of "30 micrograms" is meant a target fine particle dose ranging from 19.50 to 40.5 micrograms, preferably from 22.5 to 37.5 micrograms, more preferably from 25.5 to 34.5 micrograms. In the same way, the fine particle dose of "about 65 micrograms" is meant a target delivered dose ranging from 42.25 to 87.75 micrograms, preferably from 48.75 to 81.25 micrograms, more preferably from 55.25 to 74.75 micrograms. Similarly the fine particle dose of "about 130 micrograms" is meant a target delivered dose ranging from 84.5 to 175.5 micrograms, preferably from 97.5 to 162.5 micrograms, more preferably 110.5 to 149.50 micrograms.

In the same way, the metered nominal dose of "about 85 micrograms" is meant a target metered nominal dose ranging from 55.25 to 114.75 micrograms, preferably from 63.75 to 106.25 micrograms, more preferably from 72.25 to 97.75 micrograms. Similarly, the metered nominal dose of "about 170 micrograms" is meant a target metered nominal dose ranging from 110.50 to 229.50 micrograms, preferably from 127.50 to 212.50 micrograms, more preferably from 144.50 to 195.50 micrograms. Similarly the metered nominal dose of "about 340 micrograms" is meant a target metered nominal dose ranging from 221 to 459 micrograms, preferably from 255 to 425 micrograms, more preferably from 289 to 391 micrograms.

Thus in a preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 0.365 to 0.759 micrograms of free base abediterol together with a delivered dose ranging from 49.40 to 102.60 micrograms of mometasone furoate, preferably a delivered dose ranging from 0.421 to 0.703 micrograms of free base abediterol together with a delivered dose ranging from 57 to 95 micrograms of mometasone furoate, more preferably a delivered dose ranging from 0.477 to 0.647 micrograms of free base abediterol together with a delivered dose ranging from 64.6 to 87.4 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 0.365 to 0.759 micrograms of free base abediterol together with a delivered dose ranging from 97.50 to 202.50 micrograms of mometasone furoate, preferably a delivered dose ranging from 0.421 to 0.703 micrograms of free base abediterol together with a delivered dose ranging from 112.50 to 187.50 micrograms of mometasone furoate, more preferably a delivered dose ranging from 0.477 to 0.647 micrograms of free base abediterol together with a delivered dose ranging from from 127.50 to 172.50 micrograms of mometasone furoate.

Typically, the invention also provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 0.365 to 0.759 micrograms of free base abediterol together with a delivered dose ranging from 195 to 405 micrograms of mometasone furoate, preferably a delivered dose ranging from 0.421 to 0.703 micrograms of free base abediterol together with a delivered dose ranging from 225 to 375 micrograms of mometasone furoate, more preferably a delivered dose ranging from 0.477 to 0.647 micrograms of free base abediterol together with a delivered dose ranging from 255 to 345 micrograms of mometasone furoate.

In a preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 0.71 to 1.49 micrograms of free base abediterol together with a delivered dose ranging from 49.40 to 102.60 micrograms of mometasone furoate, preferably a delivered dose ranging from 0.82 to 1.38 micrograms of free base abediterol together with a delivered dose ranging from 57 to 95 micrograms of mometasone furoate, more preferably a delivered dose ranging from 0.93 to 1.27 micrograms of free base abediterol together with a delivered dose ranging from 64.6 to 87.4 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 0.71 to 1.49 micrograms of free base abediterol together with a delivered dose ranging from 97.50 to 202.50 micrograms of mometasone furoate, preferably a delivered dose ranging from 0.82 to 1.38 micrograms of free base abediterol together with a delivered dose ranging from 112.50 to 187.50 micrograms of mometasone furoate, more preferably a delivered dose ranging from 0.93 to 1.27 micrograms of free base abediterol together with a delivered dose ranging from 127.50 to 172.50 micrograms of mometasone furoate.

Typically, the invention also provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 0.71 to 1.49 micrograms of free base abediterol together with a delivered dose ranging from 195 to 405 micrograms of mometasone furoate, preferably a delivered dose ranging from 0.82 to 1.38 micrograms of free base abediterol together with a delivered dose ranging from 225 to 375 micrograms of mometasone furoate, more preferably a delivered dose ranging from 0.93 to 1.27 micrograms of free base abediterol together with a delivered dose ranging from 255 to 345 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 1.49 to 3.11 micrograms of free base abediterol together with a delivered dose ranging from 49.40 to 102.60 micrograms of mometasone furoate, preferably a delivered dose ranging from 1.72 to 2.88 micrograms of free base abediterol together with a delivered dose ranging from 57 to 95 micrograms of mametasone furoate, more preferably a delivered dose ranging from 1.95 to 2.65 micrograms of free base abediterol together with a delivered dose ranging from 64.6 to 87.4 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 1.49 to 3.11 micrograms of free base abediterol together with a delivered dose ranging from 97.50 to 202.50 micrograms of mometasone furoate, preferably a delivered dose ranging from 1.72 to 2.88 micrograms of free base abediterol together with a delivered dose ranging from 112.50 to 187.50 micrograms of mometasone furoate, more preferably a delivered dose ranging from 1.95 to 2.65 micrograms of free base abediterol together with a delivered dose ranging from 127.50 to 172.50 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 1.49 to 3.11 micrograms of free base abediterol together with a delivered dose ranging from 195 to 405 micrograms of mometasone furoate, preferably a delivered dose ranging from 1.72 to 2.88 micrograms of free base abediterol together with a delivered dose ranging from 225 to 375 micrograms of mometasone furoate, more preferably a delivered dose ranging from 1.95 to 2.65 micrograms of free base abediterol together with a delivered dose ranging from 255 to 345 micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.14 to 0.30 micrograms of free base abediterol together with a fine particle dose ranging from 19.50 to 40.5 micrograms of mometasone furoate, preferably a fine particle dose ranging from 0.16 to 0.28 micrograms of free base abediterol together with a fine particle dose ranging from 22.5 to 37.5 micrograms of mometasone furoate, more preferably a fine particle dose ranging from 0.18 to 0.26 micrograms of free base abediterol together with a fine particle dose ranging from 25.5 to 34.5 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.14 to 0.30 micrograms of free base abediterol together with a fine particle dose ranging from 42.25 to 87.75 micrograms of mometasone furoate, preferably a fine particle dose ranging from 0.16 to 0.28 micrograms of free base abediterol together with a fine particle dose ranging from 48.75 to 81.25 micrograms of mometasone furoate, more preferably a fine particle dose ranging from 0.18 to 0.26 micrograms of free base abediterol together with a fine particle dose ranging from 55.25 to 74.75 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.14 to 0.30 micrograms of free base abediterol together with a fine particle dose ranging from 84.5 to 175.5 micrograms of mometasone furoate, preferably a fine particle dose ranging from 0.16 to 0.28 micrograms of free base abediterol together with a fine particle dose ranging from 97.5 to 162.5 micrograms of mometasone furoate, more preferably a fine particle dose ranging from 0.18 to 0.26 micrograms of free base abediterol together with a fine particle dose ranging from 110.5 to 149.50 micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.29 to 0.61 micrograms of free base abediterol together with a fine particle dose ranging from 19.50 to 40.5 micrograms of mometasone furoate, preferably a fine particle dose ranging from 0.33 to 0.57 micrograms of free base abediterol together with a fine particle dose ranging from 22.5 to 37.5 micrograms of mometasone furoate, more preferably a fine particle dose ranging from 0.38 to 0.52 micrograms of free base abediterol together with a fine particle dose ranging from 25.5 to 34.5 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.29 to 0.61 micrograms of free base abediterol together with a fine particle dose ranging from 42.25 to 87.75 micrograms of mometasone furoate, preferably a fine particle dose ranging from 0.33 to 0.57 micrograms of free base abediterol together with a fine particle dose ranging from 48.75 to 81.25 micrograms of mometasone furoate, more preferably a fine particle dose ranging from 0.38 to 0.52 micrograms of free base abediterol together with a fine particle dose ranging from55.25 to 74.75 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.29 to 0.61 micrograms of free base abediterol together with a fine particle dose ranging from 84.5 to 175.5 micrograms of mometasone furoate, preferably a fine particle dose ranging from 0.33 to 0.57 micrograms of free base abediterol together with a fine particle dose ranging from 97.5 to 162.5 micrograms of mometasone furoate, more preferably a fine particle dose ranging from 0.38 to 0.52 micrograms of free base abediterol together with a fine particle dose ranging from 110.5 to 149.50 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.65 to 1.35 micrograms of free base abediterol together with a fine particle dose ranging from 19.50 to 40.5 micrograms of mometasone furoate, preferably a fine particle dose ranging from 0.75 to 1.25 micrograms of free base abediterol together with a fine particle dose ranging from 22.5 to 37.5 micrograms of mometasone furoate, more preferably a fine particle dose ranging from 0.85 to 1.15 micrograms of free base abediterol together with a fine particle dose ranging from 25.5 to 34.5 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.65 to 1.35 micrograms of free base abediterol together with a fine particle dose ranging from 42.25 to 87.75 micrograms of mometasone furoate, preferably a fine particle dose ranging from 0.75 to 1.25 micrograms of free base abediterol together with a fine particle dose ranging from 48.75 to 81.25 micrograms of mometasone furoate, more preferably a fine particle dose ranging from 0.85 to 1.15 micrograms of free base abediterol together with a fine particle dose ranging from 55.25 to 74.75 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.65 to 1.35 micrograms of free base abediterol together with a fine particle dose ranging from 84.5 to 175.5 micrograms of mometasone furoate, preferably a fine particle dose ranging from 0.75 to 1.25 micrograms of free base abediterol together with a fine particle dose ranging from 97.5 to 162.5 micrograms of mometasone furoate, more preferably a fine particle dose ranging from 0.85 to 1.15 micrograms of free base abediterol together with a fine particle dose ranging from 110.5 to 149.50 micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 0.406 to 0.844 micrograms of free base abediterol together with a metered nominal dose ranging from 55.25 to 114.75 micrograms of mometasone furoate, preferably a metered nominal dose ranging from 0.468 to 0.782 micrograms of free base abediterol together with a metered nominal dose ranging from 63.75 to 106.25 micrograms of mometasone furoate, more preferably a metered nominal dose ranging from 0.531 to 0.719 micrograms of free base abediterol together with a metered nominal dose ranging from 72.25 to 97.75 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 0.406 to 0.844 micrograms of free base abediterol together with a metered nominal dose ranging from 110.50 to 229.50 micrograms of mometasone furoate, preferably a metered nominal dose ranging from 0.468 to 0.782 micrograms of free base abediterol together with a metered nominal dose ranging from 127.50 to 212.50 micrograms of mometasone furoate, more preferably a metered nominal dose ranging from 0.531 to 0.719 micrograms of free base abediterol together with a metered nominal dose ranging from 144.50 to 195.50 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a mitered nominal dose ranging from 0.406 to 0.844 micrograms of free base abediterol together with a metered nominal dose ranging from 221 to 459 micrograms of mometasone furoate, preferably a metered nominal dose ranging from 0.468 to 0.782 micrograms of free base abediterol together with a metered nominal dose ranging from 255 to 425 micrograms of mometasone furoate, more preferably a metered nominal dose ranging from 0.531 to 0.719 micrograms of free base abediterol together with a metered nominal dose ranging from 289 to 391 micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 0.71 to 1.49 micrograms of free base abediterol together with a metered nominal dose ranging from 55.25 to 114.75 micrograms of mometasone furoate, preferably a metered nominal dose ranging from 0.93 to 1.57 micrograms of free base abediterol together with a metered nominal dose ranging from 63.75 to 106.25 micrograms of mometasone furoate, more preferably a metered nominal dose ranging from 1.06 to 1.44 micrograms of free base abediterol together with a metered nominal dose ranging from 72.25 to 97.75 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 0.81 to 1.69 micrograms of free base abediterol together with a metered nominal dose ranging from 110.50 to 229.50 micrograms of mometasone furoate, preferably a metered nominal dose ranging from 0.93 to 1.57 micrograms of free base abediterol together with a metered nominal dose ranging from 127.50 to 212.50 micrograms of mometasone furoate, more preferably a metered nominal dose ranging from 1.06 to 1.44 micrograms of free base abediterol together with a metered nominal dose ranging from 144.50 to 195.50 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 0. 81 to 1.69 micrograms of free base abediterol together with a metered nominal dose ranging from 221 to 459 micrograms of mometasone furoate, preferably a metered nominal dose ranging from 0.93 to 1.57 micrograms of free base abediterol together with a metered nominal dose ranging from 255 to 425 micrograms of mometasone furoate, more preferably a metered nominal dose ranging from 1.06 to 1.44 micrograms of free base abediterol together with a metered nominal dose ranging from 289 to 391 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 1.62 to 3.38 micrograms of free base abediterol together with a metered nominal dose ranging from 55.25 to 114.75 micrograms of mometasone furoate, preferably a metered nominal dose ranging from 1.87 to 3.13 micrograms of free base abediterol together with a metered nominal dose ranging from 63.75 to 106.25 micrograms of mometasone furoate, more preferably a metered nominal dose ranging from 2.12 to 2.88 micrograms of free base abediterol together with a metered nominal dose ranging from 72.25 to 97.75 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 1.62 to 3.38 micrograms of free base abediterol together with a metered nominal dose ranging from 110.50 to 229.50 micrograms of mometasone furoate, preferably a metered nominal dose ranging from 1.87 to 3.13 micrograms of free base abediterol together with a metered nominal dose ranging from 127.50 to 212.50 micrograms of mometasone furoate, more preferably a metered nominal dose ranging from 2.12 to 2.87 micrograms of free base abediterol together with a metered nominal dose ranging from 144.50 to 195.50 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 1.62 to 3.38 micrograms of free base abediterol together with a metered nominal dose ranging from 110.50 to 229.50 micrograms of mometasone furoate, preferably a metered nominal dose ranging from 1.87 to 3.13 micrograms of free base abediterol together with a metered nominal dose ranging from 127.50 to 212.50 micrograms of mometasone furoate, more preferably a metered nominal dose ranging from 2.12 to 2.87 micrograms of free base abediterol together with a metered nominal dose ranging from 144.50 to 195.50 micrograms of mometasone furoate.

In another embodiment, the invention also provides a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 1.25 micrograms of free base abediterol together with a dose equivalent to a metered nominal dose of about 70 micrograms of mometasone furoate administered with the Genuair® inhaler. The invention also provides a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 2.5 micrograms of free base abediterol together with a dose equivalent to a metered nominal dose of about 70 micrograms of mometasone furoate administered with the Genuair® inhaler. The invention also provides a pharmaceutical composition in the form of a dry powder for inhalation comprising abediterol or a pharmaceutically acceptable salt thereof and mometasone furoate in admixture with a pharmaceutically acceptable carrier, providing upon inhalation a dose equivalent to a metered nominal dose of about 0.625 micrograms of free base abediterol together with a dose equivalent to a metered nominal dose of about 70 micrograms of mometasone furoate administered with the Genuair®) inhaler.

When administered with the Genuair® inhaler a metered nominal dose of about 70 micrograms of mometasone furoate is equivalent to about 62 micrograms delivered dose and to about 22 micrograms fine particle dose.

As mentioned before, the doses of mometasone furoate are also subjected to the same acceptable variation limits as defined by the European and US pharmacopeia. Thus the delivered dose of "about 62 micrograms" is meant a target delivered dose ranging from 40.3 to 83.7 micrograms (plus/minus 35%), preferably from 46.5 to 77.5 micrograms (plus/minus 25%), more preferably from 52.7 to 71.3 micrograms (plus/minus 15%). Thus, the fine particle dose of "27 micrograms" is meant a target fine particle dose ranging from 14.3 to 29.7 micrograms, preferably from 16.5 to 27.5 micrograms, more preferably from 18.7 to 25.3 micrograms. In the same way, the metered nominal dose of "about 70 micrograms" is meant a target metered nominal dose ranging from 45.5 to 94.5 micrograms, preferably from 52.5 to 87.5 micrograms, more preferably from 59.5 to 80.5 micrograms.

Thus in a preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 0.365 to 0.759 micrograms of free base abediterol together with a delivered dose ranging from 40.3 to 83.7 micrograms of mometasone furoate, preferably a delivered dose ranging from 0.421 to 0.703 micrograms of free base abediterol together with a delivered dose ranging from 46.5 to 77.5 micrograms of mometasone furoate, more preferably a delivered dose ranging from 0.477 to 0.647 micrograms of free base abediterol together with a delivered dose ranging from 52.7 to71.3 micrograms of mometasone furoate.

In a further preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 0.71 to 1.49 micrograms of free base abediterol together with a delivered dose ranging from 40.3 to 83.7 micrograms of mometasone furoate, preferably a delivered dose ranging from 0.82 to 1.38 micrograms of free base abediterol together with a delivered dose ranging from 46.5 to 77.5 micrograms of mometasone furoate, more preferably a delivered dose ranging from 0.93 to 1.27 micrograms of free base abediterol together with a delivered dose ranging from 52.7 to71.3 micrograms of mometasone furoate.

In a further preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a delivered dose ranging from 1.49 to 3.11 micrograms of free base abediterol together with a delivered dose ranging from 40.3 to 83.7 micrograms of mometasone furoate, preferably a delivered dose ranging from 1.72 to 2.88 micrograms of free base abediterol together with a delivered dose ranging from 46.5 to 77.5 micrograms of mometasone furoate, more preferably a delivered dose ranging from 1.95 to 2.65 micrograms of free base abediterol together with a delivered dose ranging from 52.7 to71.3 micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.14 to 0.30 micrograms of free base abediterol together with a fine particle dose ranging from 14.3 to 29.7micrograms of mometasone furoate, preferably a fine particle dose ranging from 0.16 to 0.28 micrograms of free base abediterol together with a fine particle dose ranging from 16.5 to 27.5micrograms of mometasone furoate, more preferably a fine particle dose ranging from 0.18 to 0.26 micrograms of free base abediterol together with a fine particle dose ranging from 18.7 to 25.3micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.29 to 0.61 micrograms of free base abediterol together with a fine particle dose ranging from 14.3 to 29.7micrograms of mometasone furoate, preferably a fine particle dose ranging from 0.33 to 0.57 micrograms of free base abediterol together with a fine particle dose ranging from 16.5 to 27.5micrograms of mometasone furoate, more preferably a fine particle dose ranging from 0.38 to 0.52 micrograms of free base abediterol together with a fine particle dose ranging from 18.7 to 25.3micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a fine particle dose ranging from 0.65 to 1.35 micrograms of free base abediterol together with a fine particle dose ranging from 14.3 to 29.7micrograms of mometasone furoate, preferably a fine particle dose ranging from 0.75 to 1.25 micrograms of free base abediterol together with a fine particle dose ranging from 16.5 to 27.5micrograms of mometasone furoate, more preferably a fine particle dose ranging from 0.85 to 1.15 micrograms of free base abediterol together with a fine particle dose ranging from 18.7 to 25.3 micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 0.406 to 0.844 micrograms of free base abediterol together with a metered nominal dose ranging from 45.5 to 94.5 micrograms of mometasone furoate, preferably a metered nominal dose ranging from 0.468 to 0.782 micrograms of free base abediterol together with a metered nominal dose ranging from 52.5 to 87.5 micrograms of mometasone furoate, more preferably a metered nominal dose ranging from 0.531 to 0.719 micrograms of free base abediterol together with a metered nominal dose ranging from 59.6 to 80.5 micrograms of mometasone furoate.

In another preferred embodiment, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 0.71 to 1.49 micrograms of free base abediterol together with a metered nominal dose ranging from 45.5 to 94.5 micrograms of mometasone furoate, preferably a metered nominal dose ranging from 0.93 to 1.57 micrograms of free base abediterol together with a metered nominal dose ranging from 52.5 to 87.5 micrograms of mometasone furoate, more preferably a metered nominal dose ranging from 1.06 to 1.44 micrograms of free base abediterol together with a metered nominal dose ranging from 59.6 to 80.5 micrograms of mometasone furoate.

Typically, the invention provides a pharmaceutical composition in the form of a dry powder for inhalation comprising abediterol or a pharmaceutically acceptable salt thereof and mometasone furoate in admixture with a pharmaceutically acceptable carrier, providing upon inhalation a metered nominal dose ranging from 1.62 to 3.38 micrograms of free base abediterol together with a metered nominal dose ranging from 45.5 to 94.5 micrograms of mometasone furoate, preferably a metered nominal dose ranging from 1.87 to 3.13 micrograms of free base abediterol together with a metered nominal dose ranging from 52.5 to 87.5 micrograms of mometasone furoate, more preferably a metered nominal dose ranging from 2.12 to 2.88 micrograms of free base abediterol together with a metered nominal dose ranging from 59.6 to 80.5 micrograms of mometasone furoate. Typically, in these embodiments, the pharmaceutical composition is administered once daily. The once daily dose can be administered either in the morning or in the evening, preferably in the morning.

The invention also provides a pharmaceutical composition comprising abediterol, a corticosteroid as defined above and an anticholinergic, as defined above. Most preferred corticosteroid is mometasone furoate as defined above. Most preferred anticholinergic is aclidinium bromide as defined above. These triple combinations are suitable for administration once or twice a day, preferably once a day.

### Example 1

### Example 1

Clinical phase IIa study: A randomised, single dose, double-blind, double-dummy, 6 way complete cross-over, placebo and active comparator controlled, assesses the efficacy, safety and tolerability of single doses of abediterol administered once daily by inhalation compared to placebo and active comparator in persistent asthmatic patients.

Methods: 62 patients (Adult men and women aged 18-70 years) with a clinical diagnosis of persistent asthma, as defined by the 2011 GINA guideline, for at least 6 months prior to screening and with a FEV₁ 61-85% of the predicted normal values (according to Quanjer et al. 1993) were randomised to treatment sequences comprising a single-dose administration of abediterol (at metered nominal doses of 0.313, 0.625, 1.25 and 2.5 micrograms in the Genuair® device), one administration of salbutamol (at a metered nominal dose of 400 micrograms (100 micrograms x 4 puffs) in the Ventolin Evohaler® device) and placebo (administered in both Genuair® and Ventolin Evohaler®) administered all of them by means of a dry powder inhaler. The washout period between each administration was at least 7 days.

Results: Time to peak FEV₁ at day 1 was achieved within the 4h post-dose for all abediterol doses. Abediterol dose-dependently increased peak FEV₁ on Day 1 (table 1).

**Table 1: Change from baseline in peak FEV₁) on Day 1**

| | Abediterol | | | | Salbutamol | Placebo |
|---|---|---|---|---|---|---|
| | 0.313µg | 0.625 µg | 1.25 µg | 2.5 µg | 400 µg | |
| | (n=60) | (n=60) | (n=60) | (n=61) | (n=58) | (n=59) |
| ml | 477* | 524* | 573** | 608*** | 555** | 202 |

| | | | | | | |
|---|---|---|---|---|---|---|
| *p < 0.001 vs placebo,** p < 0.05 vs 0.313 µg & placebo. *** p < 0.05 vs 0.625 & 0.313 µg & placebo | | | | | | |

At all doses, abediterol clearly increases the average peak FEV₁ compared with placebo. At doses of 1.25 and 2.5 micrograms, abediterol increased bronchodilatory effect compared with salbutamol. In addition, abediterol was well tolerated, with no dose-dependent effect on EGG, laboratory parameters or adverse events.

### Example 2

Clinical phase IIa study: A randomised, single dose, double-blind, double-dummy, 6 way complete cross-over, placebo and active comparator controlled, assesses the efficacy, safety and tolerability of single doses of abediterol administered once daily by inhalation compared to placebo and active comparator in patients with stable moderate to severe chronic obstructive pulmonary disease (COPD).

Methods: 63 patients (men and women aged ≥ 40 years) with a clinical diagnosis of stable moderate to severe COPD, as defined by the 2011 GOLD guideline, with no signs of an exacerbation within 6 weeks prior to the screening visit and with a FEV₁) 30-80% of the predicted normal values (according to Quanjer et al. 1993) were randomised to treatment sequences comprising a single-dose administration of abediterol ( at a metered nominal dose of 0.625, 2.5, 5 and 10 micrograms in the Genuair® device), one administration of indacaterol (at a metered nominal dose of 150 micrograms once daily in the Onbrez Breezhaler® device) and placebo (administered in both Genuair® and Onbrez Breezhaler®). The washout period between each administration was at least 7 days. Lung function measurements included (by spirometry) trough FEV₁. Trough FEV₁ is defined as a main FEV₁) value between 23 and 24h after Investigational Medicinal Product (IMP) administration.

Results: Abediterol dose-dependently increased trough FEV₁ on Day 1 (table 2).

**Table 2: Change from baseline in morning pre-dose (trough) FEV₁ at Day 2.**

| | Abediterol | | | | Indacaterol | Placebo |
|---|---|---|---|---|---|---|
| | 0.625 µg | 2.5 µg | 5 µg | 10 µg | 150 µg | |
| | (n=67) | (n=66) | (n=) | (n=) | (n=68) | (n=68) |
| ml | 66* | 168** | 198** | 223*** | 76* | -35 |

| | | | | | | |
|---|---|---|---|---|---|---|
| *p < 0.001 vs placebo . ** p < 0.001 vs 0.625 µg & indacaterol & placebo *** p < 0.05 vs 2.5 µg & 0.625 µg & indacaterol &placebo | | | | | | |

Abediterol significantly increases the average trough FEV₁ compared with placebo. The increase in FEV₁ is statistically significant at 24h for all doses. At doses of 2.5, 5 and 10 micrograms, abediterol produces greater trough FEV₁ than with indacaterol 150 micrograms dose. Strikingly, the increase in trough FEV₁ is statistically significant compared with indacaterol. At all these doses, abediterol was well tolerated, with no dose-dependent effect on ECG, laboratory parameters or adverse events. The 2.5 micrograms dose was selected to be the minimum therapeutic effective dose producing higher bronchodilatory effects when compared with indacaterol 150 micrograms.

**Conclusion:** Abediterol provides a sustained bronchodilation over time with effect lasting more than 24h for all dose levels tested. At a dose of 0.625 micrograms, abediterol achieves a similar level of bronchodilatory effect as salbutamol and indacaterol. At a dose of 1.25 and 2.5 micrograms, abediterol shows increased bronchodilatory effects when compared with salbutamol. In addition at a dose of 2.5 micrograms, abediterol has higher bronchodilatory effects when compared with indacaterol 150 micrograms. Abediterol provides good safety and tolerability profile at all doses tested with no overall clinically relevant changes on safety outcomes (HR, QTcF, glucose and potassium). Based on the efficacy and tolerability data, Abediterol 1.25 and 2.5 micrograms were selected as the investigational dose for a future long-term clinical trial for asthmatic and COPD patients. A dose of 0.625 micrograms abediterol is also of interest since it is the minimum dose having similar therapeutic effect to salbutamol and indacaterol.

## Claims

1. A pharmaceutical composition in the form of a dry powder for inhalation comprising abediterol or a pharmaceutically acceptable salt in admixture with a pharmaceutically acceptable carrier, providing upon inhalation a dose equivalent to a metered nominal dose of about 1.25 or about 2.5 micrograms of free base abediterol administered with the Genuair® inhaler.

2. A pharmaceutical composition in the form of a dry powder for inhalation comprising abediterol or a pharmaceutically acceptable salt in admixture with a pharmaceutically acceptable carrier, providing upon inhalation a metered nominal dose ranging from 0.81 to 1.69 micrograms, preferably from 0.93 to 1.57 micrograms, more preferably from 1.06 to 1.44 micrograms of free base abediterol.

3. A pharmaceutical composition in the form of a dry powder for inhalation comprising abediterol or a pharmaceutically acceptable salt in admixture with a pharmaceutically acceptable carrier, providing upon inhalation a metered nominal dose ranging from 1.62 to 3.38 micrograms, preferably from 1.87 to 3.13 micrograms, more preferably from 2.12 to 2.88 micrograms of free base abediterol.

4. A pharmaceutical composition in the form of a dry powder for inhalation comprising abediterol or a pharmaceutically acceptable salt in admixture with a pharmaceutically acceptable carrier, providing upon inhalation a delivered dose ranging from 0.71 to 1.49 micrograms, preferably from 0.82 to 1.38 micrograms, more preferably from 0.93 to 1.27 micrograms of free base abediterol.

5. A pharmaceutical composition in the form of a dry powder for inhalation comprising abediterol or a pharmaceutically acceptable salt in admixture with a pharmaceutically acceptable carrier, providing upon inhalation a delivered dose ranging from 1.49 to 3.11 micrograms, preferably from 1.72 to 2.88, more preferably from 1.95 to 2.65 of free base abediterol.

6. A pharmaceutical composition in the form of a dry powder for inhalation comprising abediterol or a pharmaceutically acceptable salt in admixture with a pharmaceutically acceptable carrier, providing upon inhalation a fine particle dose ranging from 0.29 to 0.61 micrograms, preferably, from 0.33 to 0.57 micrograms being most preferably from 0.38 to 0.52 micrograms of free base abediterol.

7. A pharmaceutical composition in the form of a dry powder for inhalation comprising abediterol or a pharmaceutically acceptable salt in admixture with a pharmaceutically acceptable carrier, providing upon inhalation a fine particle dose ranging from 0.65 to 1.35 micrograms, preferably, from 0.75 to 1.25 micrograms being most preferably from 0.85 to 1.15 micrograms of free base abediterol.

8. The pharmaceutical composition according to any of the preceding claims, in the form of a single-dose dry powder formulation comprising a single dose of free base abediterol as defined in any one of claims 1 to 7.

9. The pharmaceutical composition according to any of the preceding claims in the form of a multi-dose dry powder formulation for administration in a mutlidose dry powder inhaler device calibrated to provide a dose of free base abediterol as defined in any one of claims 1 to 7.

10. The pharmaceutical composition according to any of the preceding claims, wherein the pharmaceutically acceptable salt is heminapadisylate.

11. The pharmaceutical composition according to any of the preceding claims wherein the pharmaceutically acceptable carrier is lactose particles.

12. The pharmaceutical composition according to any of the preceding claims wherein the ratio by weight of abediterol to carrier is from 1:1000 to 1:40000.

13. The pharmaceutical composition according to claim 12 wherein the ratio by weight of abediterol to carrier is from 1:2000 to 1:20000.

14. The pharmaceutical composition according to any of the preceding claims wherein the average particle diameter of abediterol heminapadisylate is within 1.5-5 µm.

15. The pharmaceutical composition according to any of the preceding claims wherein the carrier particles have a d10 of 90 - 160 µm, a d50 of 170 - 270 µm, and d90 of 290 - 400 µm.

16. The pharmaceutical composition according to claim 15, wherein the carrier particles are admixed with a additional particles of lactose having a d10 of 2 - 4 µm, a d50 of 7 — 10 µm, and d90 of 15 - 24 µm.

17. The pharmaceutical composition according to any of the preceding claims further comprising an effective amount of one or more additional active agents selected from M3 antagonists, PDE IV inhibitors, and corticosteroids.

18. The pharmaceutical composition according to claim 17 wherein the additional active agent is selected from tiotropium, aclidinium, mometasone, fluticasone and roflumilast, in free or pharmaceutically acceptable salt form.

19. The pharmaceutical composition according to claim 1, wherein the additional active ingredient is mometasone furoate in a dose equivalent to a metered nominal dose of about 50-900 micrograms administered with the Genuair® inhaler.

20. The pharmaceutical composition according to claim 19, wherein the mometasone furoate is present in an amount of about 70 micrograms per metered nominal dose.

21. The pharmaceutical composition according to claim 19, wherein the mometasone furoate is present in an amount of about 85 micrograms per metered nominal dose.

22. The pharmaceutical composition according to claim 19, wherein the mometasone furoate is present in an amount of about 170 micrograms per metered nominal dose.

23. The pharmaceutical composition according to claim 19, wherein the mometasone furoate is present in an amount of about 340 micrograms per metered nominal dose.

24. A method of treating a respiratory condition selected from asthma and chronic obstructive pulmonary disease in a patient in need of such treatment, comprising administering a dose of free base abediterol as defined in any one of claims 1 to 16.

25. The method according to claim 24, further comprising an effective amount of one or more additional active agents as defined in any of claims 17 to 23.

26. The use of abediterol in the manufacture of a medicament for administration in accordance with the method of claim 24 or 25.

27. The use of abediterol in the manufacture of a pharmaceutical composition according to any of claims 1-23.

28. Abediterol in free or pharmaceutically acceptable salt form for use in any of the methods of claim 24 or 25.
